Europäisches Patentamt

European Patent Office

Office européen des brevets

(11) Publication number: 0 288 937 A2

# EUROPEAN PATENT APPLICATION

(21) Application number: 88106525.4

(22) Date of filing: 22.04.88

(51) Int. Cl.4: A61M 16/06

The title of the invention has been amended (Guidelines for Examination in the EPO, A-III, 7.3).

(30) Priority: 30.04.87 US 44152

(43) Date of publication of application:
02.11.88 Bulletin 88/44

(84) Designated Contracting States:
AT BE CH DE ES FR GB GR IT LI NL SE

(71) Applicant: CAVAN CORPORATION
1064 East 400 South
Salt Lake City Utah 84102(US)

(72) Inventor: Sivill, William James
2710 E. Otero Place No. 6
Littleton Colorado 80122(US)
Inventor: Roush, Craig Robert
1064 East 400 South
Salt Lake City Utah 84102(US)
Inventor: Faddis, Chris Guy
4221 South Mark Read St.
West Valley City Utah 84119(US)

(74) Representative: Patentanwälte Grünecker, Kinkeldey, Stockmair & Partner
Maximilianstrasse 58
D-8000 München 22(DE)

(54) Disposable breathing mask.

(57) A disposable facemask includes a generally oblong annular face seal for placement against the face of a person to encircle the person's nose and mouth. The seal is made of a soft, compliant and resilient closed cell foam and is formed with an inwardly facing slot which extends along the inner wall of the seal near a front side thereof. The facemask also includes a generally oblong face plate formed of a thin sheet of flexible plastic material which includes a convex central section and an outwardly projecting rim which circumscribes the central section and which is formed to fit within the slot of the face seal at the top for receiving a person's nose, a pair of lobes which project rearwardly and outwardly from opposing sides of the face seal near the top thereof to contact and seal against the sides of the person's nose, and a beveled back rim to provide compliancy to better conform to the contours of the person's face.

FIG. I

## DISPOSABLE FACEMASK

This invention relates to a disposable facemask for use in supplying anesthesia, medication, oxygen or the like to a patient.

Masks for placement over a patient's face for purposes of sealingly supplying gas or medication to the patient are well known. Facemasks, as they are called, may take a variety of shapes but typically they consist of two parts: an annular cup for placement against the face, and some type of substantially rigid connector plate to which tubes or hoses may be connected for directing medication or anesthesia to the nose and mouth of the patient. For example, currently used facemasks employ a bladder-like cup made, for example, of silicon, and having a generally circular cross-section. The connector plate is typically made of rigid plastic, such as polystyrene, which is fitted and held in place in the cup.

Problems with the above-described prior art facemasks include inability to develop a secure seal over the patient's face to prevent escape of medication or anesthesia, and the difficulty of maintaining the facemask in a sterile condition for successive uses--the cost of manufacturing the facemask is sufficiently high that it is generally necessary to use and reuse the facemask in order to realize maximum economic benefit.

It is an object of the invention to provide a new and improved facemask design which is inexpensive to manufacture and thus may be considered disposable.

. It is another object of the invention to provide a facemask suitable for supplying anesthesia, medication, oxygen or the like to the nose and mouth of a patient.

It is a further object of the invention to provide such a facemask which, when placed against a person's face over the nose and mouth, develops a tight, leak-free seal.

The above and other objects of the invention are realized in a specific illustrative embodiment of a facemask which includes a generally oblong, annular face seal for placement against the face of a person to encircle the person's nose and mouth. The face seal is made of a resilient closed-cell foam and is formed with an inwardly facing slot which extends along the inner wall of the seal near a front side thereof. The facemask also includes a generally oblong face plate formed of a thin sheet of material which includes a convex central section and an outwardly projecting rim which circumscribes the central section and which is dimensioned to fit within the slot of the face seal, around the entire perimeter thereof.

In accordance with one aspect of the invention, the rear side of the face seal is formed with a beveled edge which flares outwardly to accommodate the contours of the face. Also, tale edge of the rear side of the face seal is formed to include a pair of lobes which project rearwardly and outwardly from opposing sides of the face seal near the top thereof to contact and seal against the sides of a person's nose when the facemask is placed against the face of the person.

In the drawings:

The above and other objects, features and advantages of the invention will become apparent from a consideration of the following detailed description presented in connection with the accompanying drawings in which:

FIG. 1 is a rear perspective view of a facemask made in accordance with the principles of the present invention;

FIG. 2 is a rear elevational view of the facemask of FIG. 1;

FIG. 3 is a top plan view of the facemask;

FIG. 4 is a front, elevational view of the facemask.

FIG. 5 is a side, elevational view of the facemask.

Referring now to the drawings:

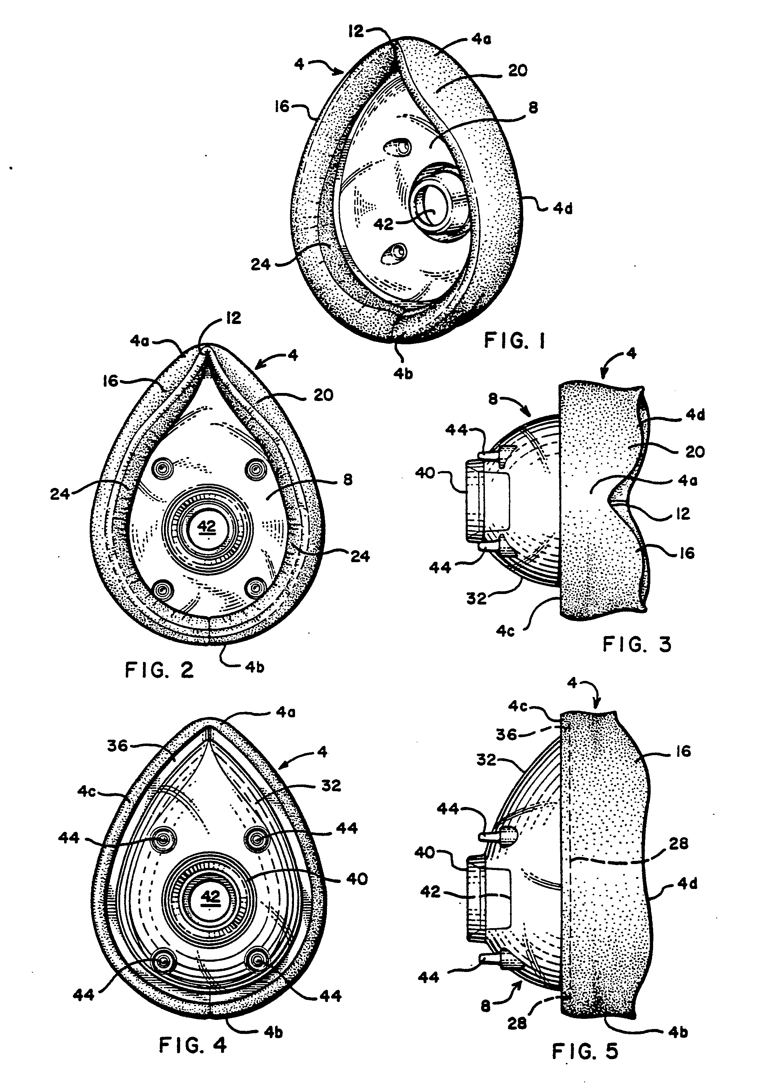

Referring to the drawings there is shown a facemask made in accordance with the present invention to include two parts: a cup or face seal 4 for placement against the face of the person on whom the facemask is to be used, and a face plate 8 fitted in the face seal and adapted to connect to hoses or other apparatus for receiving anesthesia, medication, oxygen, etc. The face seal 4 is formed from a piece or pieces of closed cell foam (each cell of the foam has skin around it so that there is no communication between the cells) into a generally oblong, annular cup. As best seen in FIGS. 1, .2, and 4, the face seal is narrower at the top 4a than it is at the bottom 4b, with the top terminating generally in a point and the bottom being rounded. The face seal is dimensioned to encircle the nose and/or mouth of a person on whom it is used, with the nose fitting in at the top (see FIG. 1) and the chin resting against the bottom.

A front rim or lip 4c of the face seal 4 lies generally in a plane as best seen in FIGS. 3 and 4. A back rim or lip 4d of the face seal is contoured to fit a wearer's face contours. For this reason, a V-shaped slot 12 is formed in the back rim 4d at the top of the face seal (FIG. 3) to receive the wearer's nose. A pair of lobes 16 and 20 project rearwardly from the back rim, beginning at the slot 12, to fit snugly on each side of the wearer's nose. The rounded contour of the back rim 4d of the face seal 4 accommodates and fits snugly against the wearer's chin.

The closed cell foam is selected to be soft, compliant and resilient to better accommodate variant facial contours of persons on whom the facemask would be used. This compliancy is further facilitated by providing a beveled edge 24 (FIGS. 1 and 2) on the inside of the back rim 4d of the face seal. This beveled edge extends around the perimeter of the back rim as shown. Also, the back rim 4d is formed to flare slightly outwardly as best seen in FIGS. 3 and 5 to again provide better compliance and thus a better seal with a person's face. Utilization of a closed cell foam material for the face seal also assists in providing a better seal since it is more difficult for gas or air to move past the seal since adjacent cells are not in communication.

Formed near the front rim 4c of the face seal is an inwardly facing slot 28 (FIGS. 1 and 5) which, as will be explained momentarily, is for receiving a rim of the face plate. This slot 28 extends around the entire interior surface of the face seal and is normally closed, but is formed to separate when the rim of the face plate is inserted therein.

The face plate 8 is a single piece of thin, transparent (about .635 millimeters) piece of plastic or similar material formed to include a generally convex central section 32 and an outwardly projecting rim 36. The rim 36 lies generally in a plane and is formed to extend about the entire perimeter of the face plate and to fit within the slot 28 formed in the face seal. When the rim 36 is inserted into the slot 28, the sides of the slot are designed to press against the rim to provide an airtight seal. The face plate 8 also includes a forwardly projecting, annular ridge 40 with an opening 42 (leading to the interior of the facemask) formed within the ridge. Also formed on the surface of the face plate 8 are four projections 44. The ridge 40 and opening 42 are provided to allow connection to a hose or other apparatus for conveying anesthesia, medication, oxygen and the like to the interior of the facemask. Projections 44 are provided to restrain the arms of a head strap to affix the facemask.

In the manner described, a simple, easy to construct facemask is provided for use in conveying medications or oxygen to a patient. The provision of a face seal made of soft, compliant and resilient closed cell foam provides a tight and yet comfortable seal on a person's face. The contouring of the back rim of the face seal with a V-shaped upper slot, rearwardly projecting lobes, and a beveled rim combines to further enhance sealing about the patient's nose and mouth. Because of the low cost of the facemask, it can be employed as a disposable article.

It is to be understood that the above-described arrangement is only illustrative of the application of the principles of the present invention. Numerous modifications and alternative arrangements may be devised by those skilled in the art without departing from the spirit and scope of the present invention and the appended claims are intended to cover such modifications and arrangements.

## Claims

1. A facemask for placement over a person's nose and mouth comprising

a generally oblong, annular face seal for placement against the face of the person to encircle the nose and mouth, said face seal being made of resilient closed cell foam, and being formed with an inwardly facing slot which extends along the inner wall of the seal near a front side thereof, and formed with a beveled edge on a rear side thereof, and

a generally oblong face plate formed of a thin sheet of material which includes a convex central section and an outwardly projecting rim which circumscribes the central section and which is dimensioned to fit within the slot of the face seal around the entire perimeter of the rim.

2. A facemask as in Claim 1 wherein the edge of the rear side of the face seal is formed to include a pair of lobes which project rearwardly from opposing sides of the face seal near the top thereof to contact and seal against the sides of the person's nose.

3. A facemask as in Claim 2 wherein said beveled edge is formed to flare outwardly substantially about the perimeter thereof.

4. A facemask as in Claim 3 wherein said material is transparent, flexible plastic.

. 5. A facemask as in Claim 4 wherein said material is about .635 millimeters in thickness.

6. A facemask as in Claim 1 wherein the face seal is formed to be narrower and to terminate generally in a point at the top, and to be broader and rounded at the bottom.

FIG. 1

FIG. 2

FIG. 3

FIG. 4

FIG. 5